# DEMANDE DE BREVET EUROPEEN

(11) **EP 0 671 375 A1**
(43) Date de publication de la demande: **13.09.1995**
(21) Numéro de dépôt: 95400400.8
(22) Date de dépôt: 27.02.1995
(51) Int. Cl.: C07C 22/08, C07C 29/40, C07C 33/46

(54) **Nouveau procédé de préparation de l'alcool 2,6-bis(trifluorométhyl)benzylique**

(30) Priorité: 04.03.1994 FR 9402516
(71) Demandeur: Hoechst Schering AgrEvo S.A., F-75020 Paris (FR)
(72) Inventeur: Babin, Didier, F-78180 Mintigny (FR); Demoute, Jean-Pierre, F-93360 Neuilly Plaisance (FR); Mackiewicz, Philippe, F-93190 Livrey Gargan (FR); Richard, Christian, F-93110 Rosny sous Bois (FR)
(74) Mandataire: Tonnellier, Marie-José

(57) **Abrégé**

L'invention a pour objet un procédé de préparation de l'alcool 2,6-bis(trifluorométhyl) benzylique, caractérisé en ce que l'on soumet l'anion A du 2,6-bis(trifluorométhyl) benzène : 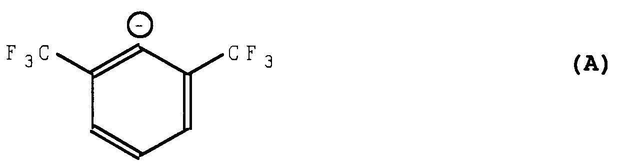
à l'action du formaldéhyde ou d'un générateur de formaldéhyde pour obtenir le produit recherché.

Le procédé de l'invention trouve son utilisation dans la synthèse de produits agrochimiques.

## Description

La présente invention concerne un nouveau procédé de préparation de l'alcool 2,6-bis(trifluorométhyl) benzylique.

L'invention a pour objet un procédé de préparation de l'alcool 2,6-bis(trifluorométhyl) benzylique, caractérisé en ce que l'on soumet l'anion A du 2,6-bis(trifluorométhyl) benzène : 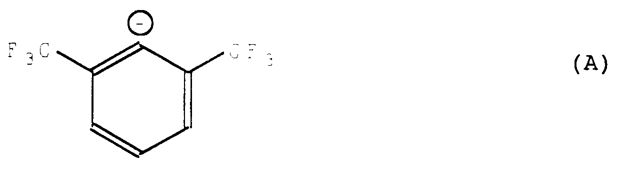
à l'action du formaldéhyde ou d'un générateur de formaldéhyde pour obtenir le produit recherché.

L'alcool 2,6-bis(trifluorométhyl) benzylique est un produit connu décrit et revendiqué dans la demande de brevet européen 0557192.

Le procédé décrit dans cette demande de brevet consiste à faire réagir le sulfate de diméthyle sur l'acide 2,6-bis(trifluorométhyl) benzoïque pour obtenir le 2,6-bis(trifluorométhyl) benzoate de méthyle que l'on soumet à l'action de l'hydrure de diisobutylaluminium (DIBAH) pour obtenir le produit recherché.

Le procédé décrit dans la demande de brevet précité peut donc être schématisé comme suit :

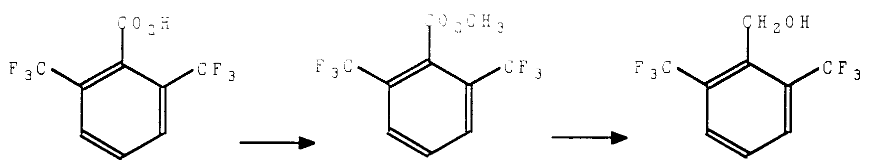

La préparation de l'acide 2,6-bis(trifluorométhyl) benzoïque utilisé au départ du procédé peut être obtenu à partir du 2,6-bis(trifluorométhyl) benzène comme décrit dans la littérature (cf par exemple Schlosser et coll. ; Synlett Décembre 1990, p. 747 ou Grocock et coll., J. Chem. Soc. (C), 1971, p. 3305).

On vient de découvrir un nouveau procédé de préparation de l'alcool 2,6-bis(trifluorométhyl) benzylique à partir du 2,6-bis(trifluorométhyl) benzène rapide et facilement industrialisable.

Le 2,6-bis(trifluorométhyl) benzène utilisé comme produit de départ est un produit commercial. Les avantages du procédé apparaitront clairement à la lecture de la partie expérimentale exposée ci-après.

Le procédé de l'invention qui consiste en une hydroxyméthylation directe de l'anion, formé "in situ", au moyen du formaldéhyde évite l'utilisation de l'acide 2,6-bis(trifluorométhyl) benzoïque.

Le procédé de l'invention est donc plus court que le procédé connu à ce jour. De plus, le procédé de l'invention permet d'obtenir un bon rendement en alcool 2,6-bis(trifluorométhyl) benzylique, alors que les 3 alcools : 2,4-bis(trifluorométhyl) benzylique, 2,6-bis(trifluorométhyl) benzylique et 3,5-bis(trifluorométhyl) benzylique étaient susceptibles d'être obtenus

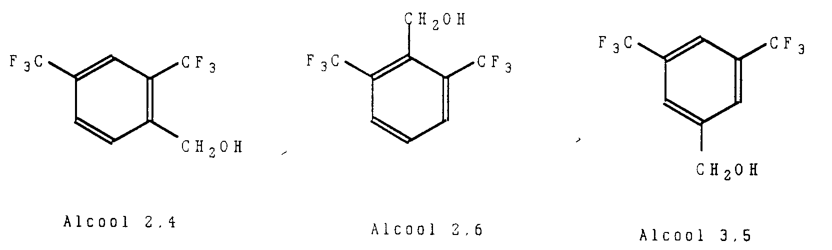

L'invention a notamment pour objet un procédé caractérisé en ce que l'on fait réagir l'anion A défini ci-dessus avec le formaldéhyde ou encore un procédé caractérisé en ce que l'on fait réagir l'anion A défini ci-dessus avec un générateur de formaldéhyde choisi dans le groupe constitué par les polyformaldéhydes, l'hexaméthylènetétramine, la rongalite et le chlorure de méthoxyéthoxyméthyle.

Comme polyformaldéhydes, on peut citer le paraformaldéhyde, le dioxanne, le polyoxyméthylène®.

L'invention a spécialement pour objet un procédé caractérisé en ce que l'anion A défini ci-dessus est obtenu en faisant réagir une base ou un mélange de bases sur le 2,6-bis(trifluorométhyl) benzène.

L'invention a plus spécialement pour objet un procédé caractérisé en ce que l'on utilise comme base un dérivé lithié ou un mélange de dérivés lithiés R-Li dans lequel R représente un radical alkyle ou aralkyle renfermant jusqu'à 8 atomes de carbone, en présence éventuellement d'un agent activant.

R peut être par exemple un radical méthyle, éthyle, propyle, n-butyle, sec-butyle, terbutyle, hexyle, 2-éthylhexyle ou 4,4'-ditertbutylphényle.

L'invention a tout spécialement pour objet un procédé de préparation caractérisé en ce que le dérivé lithié répond à la formule R-Li dans laquelle R est un radical méthyle ou n-butyle ou ter-butyle.

L'invention a également particulièrement pour objet un procédé caractérisé en ce que l'agent activant est un alcoolate alcalin ou alcalino-terreux.

L'alcoolate utilisé peut être un alcoolate de lithium, de sodium, de potassium, par exemple un méthylate, un terbutylate, un n-butylate, un 3-méthyl 3-pentylate, un ter-heptylate ou un octodylate.

L'invention a tout particulièrement pour objet un procédé caractérisé en ce que l'alcoolate utilisé est un terbutylate ou un teramylate de lithium, de sodium ou de potassium.

Parmi les procédés préférés de l'invention, on peut citer le procédé caractérisé en ce que l'on soumet l'anion A à l'action de butyllithium en présence de téramylate de potassium ou encore le procédé caractérisé en ce que l'on soumet l'anion A à un mélange de méthyllithium et de terbutylate de potassium.

L'invention a également pour objet, un procédé caractérisé en ce que la base utilisée est une base organosodée ou organopotassée en présence éventuellement d'un agent activant.

Pour remplacer les bases lithiées, on peut ainsi utiliser le butyl sodium, l'amylsodium ; ces bases peuvent être préparées "in situ" par action de sodium ou de potassium et de chlorure d'alkyle selon les procédés décrits par exemple par Morton JASC, 75 (1953), 134 ou Bryce-Smith, J. Chem. Soc. 1954, 1079.

L'agent activant est de préférence un alcoolate de métal alcalin ou alcalino-terreux.

Les alcoolates de métal alcalin ou alcalino-terreux sont de préférence les alcoolates mentionnés ci-dessus.

L'invention a tout spécialement pour objet un procédé caractérisé en ce que l'on opère à une température comprise entre -10°C et -90°C, notamment un procédé caractérisé en ce que l'on opère à la température de -70°C ± 5°C.

On peut utiliser différents solvants organiques. De très bons résultats ont été obtenus avec le diglyme, le diméthyléther de l'éthylène glycol et le tétrahydrofuranne.

L'invention a également pour objet, à titre de produit chimique nouveau l'anion A, défini ci-dessus.

Les exemples suivants illustrent l'invention.

### EXEMPLE 1 : alcool 2,6-bis(trifluorométhyl) benzylique

On introduit 19,94 g de téramylate de sodium dans 250 ml de tétrahydrofuranne. On maintient le mélange réactionnel sous agitation à 20°C ± 1°C, jusqu'à l'obtention d'une solution que l'on refroidit ensuite à -60°C ± 2°C. On ajoute sous agitation à cette température 31,15 g de 2,6-bis(trifluorométhyl) benzène. On introduit ensuite en 2 h 30 minutes, entre -58°C et -62°C, 100 ml d'une solution de butyllithium à 15 % dans l'hexane. On agite encore pendant 30 minutes à - 60°C ± 2°C et introduit en une seule fois 21,83 g de paraformaldéhyde. On maintient le mélange réactionnel sous agitation pendant 30 minutes à -60°C ± 2°C puis porte la température à +15°C ± 2°C. On verse le mélange réactionnel dans un mélange de glace et de solution aqueuse d'acide chlorhydrique. On agite pendant 16 heures à 20°C, décante et réextrait à l'hexane. On joint les phases organiques, et concentre sous pression réduite (sous une pression supérieure ou égale à 20 millibars et à une température extérieure inférieure ou égale à 50°C. On obtient 39,9 g de produit brut que l'on distille à nouveau. On obtient ainsi 17,25 g d'alcool 2,6-bis(trifluorométhyl) benzylique. F = 58°C.

### EXEMPLE 2 : alcool 2,6-bis(trifluorométhyl) benzylique

On introduit 18 g de terbutylate de potassium dans 200 ml de tétrahydrofuranne. On refroidit la solution obtenue à -60°C et ajoute 24,80 g de 2,6-bis(trifluorométhyl) benzène dans 20 ml de tétrahydrofuranne. On ajoute ensuite, en maintenant la température à -60°C, 80 ml d'une solution 1,6 M de méthyllithium dans l'éther. On maintient le mélange réactionnel à -60°C pendant 3 heures et laisse la température remonter à -40°C, et ajoute à -40°C 300 ml d'une solution de formaldéhyde 0,3 M dans le tétrahydrofuranne. On ajoute 1,7 g de formaldéhyde en branche. On maintient le mélange réactionnel 1 h 30 à -20°C et laisse la température du mélange remonter à la température ambiante. On verse sur une solution d'acide chlorhydrique 2N et agite 16 heures à la température ambiante. On extrait au méthylterbutyléther, lave à l'eau, sèche et distille le solvant. On chromatographie le produit obtenu sur silice en éluant avec le mélange hexane-acétate d'éthyle (9-1) et obtient ainsi 20,4 g d'un produit que l'on distille. On obtient 19,4 g de produit recherché.

### EXEMPLE 3 : alcool 2,6-(bistrifluorométhyl) benzylique

En opérant comme à l'exemple 1, mais en réalisant la préparation de l'anion A à -70°C, on obtient un rendement supérieur à celui obtenu à l'exemple 1.

### EXEMPLE 4 : alcool 2,6-bis(trifluorométhyl) benzylique

En opérant comme à l'exemple 2, mais en réalisant la préparation de l'anion A à -70°C, on obtient un rendement supérieur à celui obtenu à l'exemple 2.

## Revendications

**1)** Procédé de préparation de l'alcool 2,6-bis(trifluorométhyl) benzylique, caractérisé en ce que l'on soumet l'anion A du 2,6-bis(trifluorométhyl) benzène : 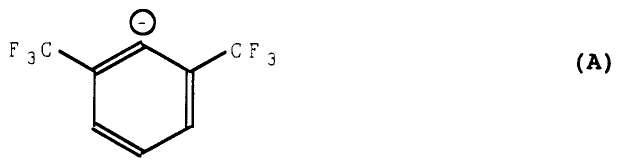 à l'action du formaldéhyde ou d'un générateur de formaldéhyde pour obtenir le produit recherché.

**2)** Procédé selon la revendication 1, caractérisé en ce que l'on fait réagir l'anion A avec le formaldéhyde.

**3)** Procédé selon la revendication 1, caractérisé en ce que l'on fait réagir l'anion A avec un générateur de formaldéhyde choisi dans le groupe constitué par les polyformaldéhydes, l'hexaméthylènetétramine, la rongalite et le chlorure de méthoxyéthoxyméthyle.

**4)** Procédé selon l'une quelconque des revendications 1 à 3, caractérisé en ce que l'anion A est obtenu en faisant réagir une base ou un mélange de bases sur le 2,6-bis(trifluorométhyl) benzène.

**5)** Procédé selon la revendication 4, caractérisé en ce que l'on utilise comme base un dérivé lithié ou un mélange de dérivés lithiés R-Li dans lequel R représente un radical alkyle ou aralkyle renfermant jusqu'à 8 atomes de carbone, en présence éventuellement d'un agent activant.

**6)** Procédé selon la revendication 5, caractérisé en ce que le dérivé lithié répond à la formule R-Li dans laquelle R est un radical méthyle ou n-butyle ou ter-butyle.

**7)** Procédé selon la revendication 5 ou 6, caractérisé en ce que l'agent activant est un alcoolate alcalin ou alcalino-terreux.

**8)** Procédé selon la revendication 7, caractérisé en ce que l'alcoolate utilisé est un terbutylate ou un teramylate de lithium, de sodium ou de potassium.

**9)** Procédé selon la revendication 8, caractérisé en ce que l'on soumet l'anion A à l'action du butyllithium en présence de téramylate de potassium.

**10)** Procédé selon la revendication 8, caractérisé en ce que l'on soumet l'anion A à un mélange de méthyllithium et de terbutylate de potassium.

**11)** Procédé selon la revendication 4, caractérisé en ce que la base utilisée est une base organosodée ou organopotassée en présence éventuellement d'un agent activant.

**12)** Procédé selon l'une quelconque des revendications 4 à 11, caractérisé en ce que l'on opère à une température comprise entre -10°C et -90°C.

**13)** Procédé selon la revendication 12, caractérisé en ce que l'on opère à la température de -70°C ± 5°C.

**14)** A titre de produit chimique nouveau, l'anion A tel que défini à la revendication 4.
